(19) Europäisches Patentamt
European Patent Office
Office européen des brevets

(11) Veröffentlichungsnummer: **0 041 927**
**B1**

(12) # EUROPÄISCHE PATENTSCHRIFT

(45) Veröffentlichungstag der Patentschrift: **25.04.84**

(51) Int. Cl.³: **C 23 F 11/12,** C 10 M 1/00

(21) Anmeldenummer: **81810225.3**

(22) Anmeldetag: **05.06.81**

(54) **Korrosionshemmende Zusammensetzungen.**

(30) Priorität: **11.06.80 GB 8019126**

(43) Veröffentlichungstag der Anmeldung: **16.12.81 Patentblatt 81/50**

(45) Bekanntmachung des Hinweises auf die Patenterteilung: **25.04.84 Patentblatt 84/17**

(84) Benannte Vertragsstaaten: **DE FR GB IT**

(56) Entgegenhaltungen:
**FR - A - 2 304 585**
**GB - A - 578 945**
**US - A - 2 820 805**
**US - A - 3 012 071**
**US - A - 3 763 231**
**US - A - 3 989 637**

(73) Patentinhaber: **CIBA-GEIGY AG, Postfach, CH-4002 Basel (CH)**

(72) Erfinder: **Clark, David Ronald, Dr., 16 Ennerdale Drive, Sale Cheshire M33 5NE (GB)**

**EP 0 041 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Korrosionshemmende Zusammensetzungen

Vorliegende Erfindung betrifft Zusammensetzungen aus einer mit Eisenmetallen in Berührung kommenden Funktionsflüssigkeit und 5-Ketocarbonsäurederivaten als Korrosionshemmstoff.

Gegenstand vorliegender Erfindung ist eine Zusammensetzung, welche dadurch gekennzeichnet ist, daß sie aus einer mit Eisenmetallen in Berührung kommenden Funktionsflüssigkeit und, als Korrosionshemmstoff, einer Verbindung der Formel

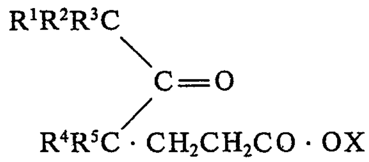

(I)

worin X für H, ein Alkali- oder Erdalkalimetall, vorzugsweise Na oder K, $NH_4$ oder den Rest eines protonierten Amins steht oder die Gruppe $-OX$ den Rest eines Alkanols mit 1—20 Kohlenstoffatomen oder eines Di-, Tri-, oder Tetraols mit 2 bis 12 Kohlenstoffatomen darstellt, $R^1$, $R^2$ und $R^4$ gleich oder verschieden sind und je für H oder $-CH_2CH_2COOX$ stehen, wobei X seine obige Bedeutung hat, $R^3$ und $R^5$ gleich oder verschieden sind und je für H oder $-CH_2CH_2COOX$ stehen, wobei X seine obige Bedeutung hat, bzw. $R^3$ oder $R^5$ eine geradkettige oder verzweigte Alkylgruppe mit 3 bis 16, vorzugsweise 7 bis 12 Kohlenstoffatomen darstellen oder $R^3$ und $R^5$ zusammen mit dem Kohlenstoffatom, an das sie gebunden sind, einen Cycloalkanonring mit 8 bis 15 Kohlenstoffatomen bilden, besteht, mit der Bedingung, daß $R^4$ und $R^5$ gleichzeitig den Rest $-CH_2CH_2COOX$ nicht bedeuten können, wenn $R^1$, $R^2$ und $R^3$ gleichzeitig Wasserstoff sind, und daß $R^5$ keine Alkylgruppe mit 3 bis 5 Kohlenstoffatomen darstellen kann, wenn $R^1$, $R^2$ und $R^3$ gleichzeitig Wasserstoff und $R^4$ der Rest $-CH_2CH_2COOX$ oder Wasserstoff sind, wobei X seine obige Bedeutung hat.

Ist X der Rest eines protonierten Amins, so kann dieser ein Alkylaminrest mit 1—20 Kohlenstoffatomen sein, z. B. Methylamin, Äthylamin, Propylamin, Butylamin, Hexylamin, Octylamin, Laurylamin, Stearylamin oder Eikosylamin, Di- oder Triäthanolamin oder ein stickstoffhaltiger Heterocyclus z. B. Morpholin.

Gruppen $-OX$ als Reste von $C_{1-20}$-Alkanolen sind unter anderem Methoxy-, Äthoxy-, Propoxy-, Butoxy-, Hexyloxy-, Octyloxy-, Nonyloxy-, Decyloxy-, Dodecyloxy-, Stearyloxy- und Eikosyloxygruppen. Infragekommende Gruppen $-OX$ als Reste eines $C_{2-12}$-Di-, Tri- oder Tetraols sind die Reste von Äthylenglykol, Propylenglykol, Diäthylenglykol, Butylenglykol, Neopentylglykol, Trimethylolpropan und Pentaerythrit.

Als Alkylgruppen $R^3$ und $R^5$ kommen die n-, sek.- und tert.-Isomeren von Propyl-, Butyl-, Amyl-, Hexyl-, Heptyl-, Octyl-, Nonyl-, Decyl-, Dodecyl-, Tetradecyl- und Hexadecylgruppen in Betracht.

Eine bevorzugte Klasse von Verbindungen der Formel I entspricht der Formel:

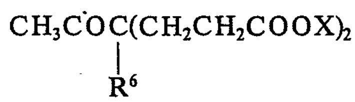

(II)

worin X seine obige Bedeutung hat und $R^6$ für eine geradkettige oder verzweigte Alkylgruppe mit 6 bis 16, vorzugsweise 7 bis 12, Kohlenstoffatomen steht.

Eine weitere bevorzugte Klasse entspricht der Formel:

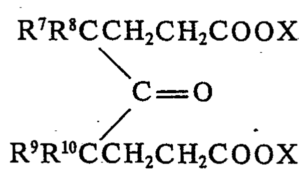

(III)

worin X seine obige Bedeutung hat, $R^7$ und $R^{10}$ gleich oder verschieden sind und je für eine geradkettige oder verzweigte Alkylgruppe mit 3 bis 10 Kohlenstoffatomen stehen sowie $R^8$ und $R^9$ gleich oder verschieden sind und je H oder $-CH_2CH_2COOX$ darstellen, wobei X seine obige Bedeutung hat.

Noch eine weitere bevorzugte Klasse entspricht der Formel:

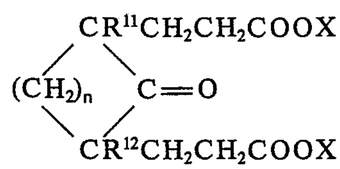

(IV)

worin X seine obige Bedeutung hat, $R^{11}$ und $R^{12}$ gleich oder verschieden sind und je für H oder $-CH_2CH_2COOX$ stehen, wobei X seine obige Bedeutung hat, und n eine ganze Zahl von 5 bis 12, vorzugsweise 9 bis 11 ist.

Spezielle Beispiele für Verbindungen der Formel I sind:
4-n-Heptyl-4-acetylpimelinsäure,
4-n-Octyl-4-acetylpimelinsäure,
4-n-Nonyl-4-acetylpimelinsäure,
4-n-Decyl-4-acetylpimelinsäure,
4-n-Dodecyl-4-acetylpimelinsäure,
4-n-Tetradecyl-4-acetylpimelinsäure,
2,2,10-Tris-(2'-carboxyäthyl)-cyclodecanon,
2,2,12-Tris-(2'-carboxyäthyl)-cyclododecanon,
2,2,12-Tris-(2'-carboxyäthyl)-cyclotridecanon,
5,7,7-Tris-(2'-carboxyäthyl)-undecanon-6,
6,8,8-Tris-(2'-carboxyäthyl)-tridecanon-7 und
8,10,10-Tris-(2'-carboxyäthyl)-heptadecanon-9 sowie
deren Salze, z. B. Na-, K- und $NH_4$-Salze, protonierte Aminanaloge und Esteranaloge, z. B. deren Mono- und Di-esteranaloge.

Die Verbindungen der Formel I sind bekannte, zusammen mit einer Herstellungsmethode beispielsweise in der britischen Patentschrift Nr. 1 490 287 beschriebene Verbindungen. Die dort erwähnte Verwendung der Verbindungen der Formel I beschränkte sich jedoch auf deren Wirkung zur Regulierung des Abbindens von Gips.

Vorzugsweise enthält die erfindungsgemäße Zusammensetzung 0,001 bis 5 Gew.-% der Verbindung der Formel I, bezogen auf das Gesamtgewicht der Zusammensetzung.

Beispiele für in den erfindungsgemäßen Zusammensetzungen zu verwendende Funktionsflüssigkeiten sind unter anderem rein wäßrige Systeme, z. B. Kühlwassersysteme, nicht-wäßrige Systeme wie Schmierstoffe auf Grundlage eines Mineralöls oder synthetischen Carbonsäureesters, Schmierfette, zeitweilige Schutzanstriche und hydraulische Flüssigkeiten auf Mineralöl- oder Phosphatester-grundlage sowie gemischte wäßrig/nicht-wäßrige Systeme, z. B. wäßrige Polyglykol/Polyglykolächergemische, Glykolsysteme, Öl-in-Wasser- und Wasser-in-Ölemulsionen, Metallbearbeitungsflüssigkeiten mit Mineralölen oder wäßrigen Systemen als Grundlage sowie Anstrichfarben auf wäßriger Grundlage.

Zur Behandlung von wäßrigen, z. B. Kühlwassersystemen bevorzugt man solche Verbindungen, welche (a) der Formel

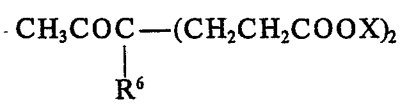

(II)

worin X seine obige Bedeutung hat und vorzugsweise für H, Na oder K steht und $R^6$ seine obige Bedeutung hat und vorzugsweise für eine Alkylgruppe mit 6 bis 16, vorzugsweise 7 bis 12, Kohlenstoffatomen und insbesondere eine Octylgruppe steht, oder deren Monocarboxyäthylanalogen oder (b) der Formel

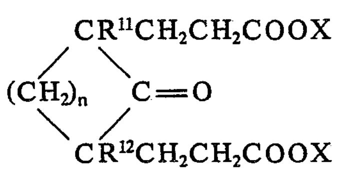

(IV)

worin X seine obige Bedeutung hat und vorzugsweise für H, Na oder K steht, $R^{11}$ H oder $-CH_2CH_2COOX$, wobei X seine obige Bedeutung hat, und vorzugsweise H darstellt, $R^{12}$ für H oder $-CH_2CH_2COOX$, wobei X seine obige Bedeutung hat, und vorzugsweise für $-CH_2CH_2COOX$ (wobei X seine obige Bedeutung hat) steht und n eine ganze Zahl von 5 bis 12, vorzugsweise 9 bis 11, ist, entsprechen.

Zur Behandlung von Metallbearbeitungsflüssigkeiten bevorzugt man solche Verbindungen, welche (a) der Formel $CH_3COC(R^6)(CH_2CH_2COOX)_2$, worin X seine obige Bedeutung hat und vorzugsweise für H, Diäthanolamin, Morpholin oder insbesondere Triäthanolamin steht und $R^6$ seine obige Bedeutung hat und vorzugsweise Octyl- oder Decylgruppe darstellt, oder deren Monocarboxyäthylanaloge, (b) der Formel

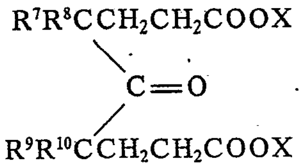

(III)

worin X seine obige Bedeutung hat und vorzugsweise für H, Diäthanolamin oder Morpholin oder insbesondere Triäthanolamin steht, $R^8$ und $R^9$ gleich oder verschieden sind und je H oder $Ch_2CH_2COOX$ darstellen, wobei X seine obige Bedeutung hat, und $R^8$ insbesondere für H steht sowie $R^7$ und $R^{10}$ gleich oder verschieden sind und je für eine Alkylgruppe mit 3—10 C Atomen und beide vorzugsweise für eine Amylgruppe stehen, oder (c) der Formel

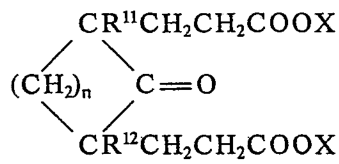

(IV)

worin X seine obige Bedeutung hat und vorzugsweise für H, Diäthanolamin, Morpholin oder, insbesondere, Triäthanolamin steht, $R^{11}$ seine obige Bedeutung hat und insbesondere H darstellt, $R^{12}$ seine obige Bedeutung hat und vorzugsweise $CH_2CH_2COOX$ bedeutet sowie n seine obige Bedeutung hat und vorzugsweise 9 bis 11 ist, entsprechen.

Zur Behandlung von Funktionsflüssigkeiten auf Ölgrundlage bevorzugt man solche Verbindungen, welche der oben definierten Formel I entsprechen, worin (a) $R^1$, $R^2$ und $R^3$ je für H stehen, $R^4$ seine obige Bedeutung hat, $R^5$ eine Alkylgruppe mit 8 bis 16 Kohlenstoffatomen darstellt und X vorzugsweise H oder ein protoniertes Amin oder, falls $R^4$ $-CH_2CH_2COOH$ ist, eine Alkyl- oder eine Hydroxyalkylgruppe bedeutet, (b) $R^1$ H ist, $R^2$ und $R^4$ entweder beide für H oder eines für H und das andere für $-CH_2CH_2COOX$ stehen, $R^3$ und $R^5$ Alkyl mit 5 bis 16, insbesondere 8 bis 16, Kohlenstoffatomen darstellen und X H oder ein protoniertes Amin oder, falls $R^2$ oder $R^4$ $-CH_2CH_2COOH$ ist, eine Estergruppe (Alkyl oder Hydroxyalkyl) bedeutet, oder (c) $R^1$ H ist, $R^2$ und $R^4$ entweder beide für H oder eines für H und das andere für $-CH_2CH_2COOX$ stehen, $R^3$ und $R^5$ die Gruppe $-(CH_2)_m-$ mit m einer ganzen Zahl von 9 bis 12 darstellen und X H oder ein protoniertes Amin oder, falls $R^2$ oder $R^4$ $-CH_2CH_2COOH$ ist, eine Estergruppe bedeutet.

In erfindungsgemäßen, rein wäßrige Funktionsflüssigkeiten enthaltenden Zusammensetzungen, z. B. erfindungsgemäßen Kühlwassersystemen, kann man Verbindungen der Formel I für sich oder im Gemisch mit weiteren Zusatzstoffen einsetzen, z. B. bekannten korrosionshemmenden Stoffen wie Phosphonaten, Phosphonocarbonsäuren sowie N-Acylsarkosinen, Imidazolidinen, Triäthanolamin und Fettaminen, und Polycarbonsäuren, wasserlöslichen Azolen, z. B. Triazolen wie Benztriazol, und weiteren kupferpassivierenden Derivaten, z. B. 2-Mercaptobenztriazol. Weitere dabei bevorzugte Zusatzstoffe sind Dispergier- und/oder Impfmittel wie zum Beispiel polymerisierte Acrylsäure und deren Salze, hydrolysiertes Polyacrylnitril, polymerisierte Methacrylsäure und deren Salze, Polyacrylamid und dessen Copolymere mit Acryl- und Methacrylsäure, Ligninsulfonsäure und deren Salze, Tannin, Naphthalinsulfonsäure/Formaldehyd-kondensationsprodukte, Stärke und deren Derivate sowie Cellulose. Spezielle Impfmittel wie zum Beispiel Alkylphosphonsäuren, 1-Aminoalkyl-1,1-diphosphonsäuren und deren Salze, Polycarbonsäuren z. B. Polymaleinsäuren, und Alkaliphosphate, lassen sich ebenfalls zusammen mit den Verbindungen der Formel I verwenden.

Ferner kann man Fällungsmittel wie Alkaliorthophosphate und -carbonate, Sauerstoffaufnehmer wie Alkalisulfite und Hydrazine, Sequestriermittel wie Nitrilotriessigsäure und deren Salze sowie Äthylendiamintetraessigsäure und deren Salze, Antischaummittel wie Distearylsebacinamid, Distearyladipamid und verwandte, durch Kondensation mit Äthylenoxyd und/oder Propylenoxyd erhaltene Produkte, neben Fettalkoholen wie Caprylalkoholen und deren Äthylenoxydkondensaten zusammen mit den Verbindungen der Formel I einsetzen.

Beispiele für in nicht-wäßrigen Funktionsflüssigkeiten mitverwendbare Zusatzstoffe sind Antioxidantien, Metallpassivatoren, Rostschutzmittel, Viskositätsindexverbesserer, Fließpunkterniedriger, Dispergiermittel, Tenside, Höchstdruckzusätze und Verschleißschutzzusätze.

Beispiele für Antioxidantien sind:

a) Gegebenenfalls alkylierte aromatische Amine und deren Gemische, z. B. Dioctyldiphenylamin, Mono-t-octylphenyl- $\alpha$- und -$\beta$-Naphthylamin, Dioctylphenothiazin, Phenyl-$\alpha$-naphthylamin und N,N'-Di-sek.-butyl-p-phenylendiamin.
b) Sterisch gehinderte Phenole z. B. 2,6-Ditertiärbutyl-p-kresol, 4,4'-Bis-(2,6-diisopropylphenol), 2,4,6-Triisopropylphenol, 2,2'-Thio-bis-(4-methyl-6-tert.-butylphenol).
c) Alkyl-, Aryl- oder Alkaryl-phosphite z. B. Triphenylphosphit, Trinonylphosphit und Diphenyldecylphosphit.

d) Ester der Thiodipropionsäure z. B. Dilaurylthiodipropionat.
e) Salze der Carbamin- und Dithiophosphorsäure z. B. Antimon-diamyldithiocarbamat und Zinkdiamyldithiophosphat.
f) Metallsalze und Komplexe mit organischen Chelatbildnern, z. B. Kupfer-bis-(trifluoracetonate), Kupferphthalocyanin und EDTA-tributylester-mononatriumsalz.
g) Radikal-antioxidantien und deren Vorstufen z. B. Nitroxyde.
h) Kombinationen von zwei oder mehr Antioxidantien aus irgendwelchen obigen Gruppen, d. h. ein alkyliertes Amin und ein sterisch gehindertes Phenol.

Metallpassivatoren sind beispielsweise:
a) für Kupfer z. B. Benztriazol, 5,5'-Methylen-bis-benztriazol, 4,5,6,7-Tetrahydrobenztriazol, 2,5-Dimercaptothiadiazol, Salicylidenpropylendiamin, Salze des Salicylaminoguanidins und Chinizarin.
b) für Magnesium z. B. Pyridylamine.
c) für Blei z. B. Sebacinsäure, Propylgallat und Chinazarin.
d) Kombinationen aus zwei oder mehreren der obigen Zusatzstoffe.

Beispiele für Rostschutzmittel sind:
a) Organische Säuren und deren Ester, Metallsalze und Anhydride, z. B. N-Oleoylsarkosin, Sorbitanmono-oleat, Bleinaphthenat und Dodecenylbernsteinsäureanhydrid.
b) Stickstoffhaltige Stoffe z. B.
   i) Primäre, sekundäre oder tertiäre aliphatische oder cycloaliphatische Amine und Aminsalze organischer und anorganischer Säuren, z. B. Morpholin, Stearylamin und Triäthanolamincaprylat.
   ii) Heterocyclische Verbindungen z. B. Imidazoline und Oxazoline.
c) Phosphorhaltige Stoffe z. B. anorganische Phosphate, Phosphonsäuren und Aminphosphate.
d) Schwefelhaltige Stoffe z. B. Barium-dinonylnaphthalinsulfonate.
e) Kombinationen von zwei oder mehreren der obigen Zusatzstoffe.

Viskositätsindexverbesserer/Fließpunkterniedriger sind beispielsweise:
z. B. Polyacrylate, Polybutene und Polyvinylpyrrolidone.

Beispiele für Dispergiermittel/Tenside sind:
z. B. Metallsulfonate (Ca, Ba, Mg) und -phenolate sowie Polybutenylsuccinimide.

Höchstdruck/Verschleißschutzzusätze sind beispielsweise:
schwefel- und/oder phosphor- und/oder halogenhaltige Stoffe z. B. sulfuriertes Spermöl, Tritolylphosphat und chlorierte Paraffine.

Bei erfindungsgemäßen Zusammensetzungen, in denen die Funktionsflüssigkeit ein gemischtes wäßrig/nicht-wäßriges System darstellt, kann man die Verbindungen der Formel I für sich oder im Gemisch mit anderen Verbindungen, z. B. bekannten Korrosionshemmstoffen und im Fall von Bearbeitungsflüssigkeiten mit einem Höchstdruckzusatz, einsetzen.

Beispiele für weitere, gegebenenfalls in den erfindungsgemäßen wäßrigen Systemen neben den Aminsalzen der Verbindungen der Formel I vorliegende Korrosionshemmstoffe sind unter anderem die folgenden Gruppen:
a) Organische Säuren und deren Ester oder Ammonium-, Amin-, Alkanolamin- und Metallsalze, zum Beispiel Benzoesäure, p-tert.-Butylbenzoesäure, Dinatriumsebacinat, Triäthanolaminlaurat, Isononansäure, p-Toluolsulfonamido-capronsäure-triäthanolaminsalz, Natrium-N-lauroylsarkosinat oder Nonylphenoxyessigsäure;
b) stickstoffhaltige Stoffe, wie solche der folgenden Typen: Fettsäurealkanolamide, Imidazoline, z. B. 1-Hydroxyäthyl-2-oleylimidazolin, Oxazoline, Triazole z. B. Benztriazole und anorganische Salze z. B. Natriumnitrit und -nitrat;
c) Phosphorhaltige Stoffe wie solche der folgenden Typen: Aminphosphate, Phosphonsäuren oder anorganische Salze, z. B. Natriumdihydrogenphosphat;
d) Schwefelhaltige Verbindungen, wie solche der folgenden Typen: Natrium-, Calcium- oder Bariumpetroleumsulfonate oder Heterocyclen z. B. Natrium-mercaptobenzthiazol.

Beispiele für gegebenenfalls in den erfindungsgemäßen Flüssigkeiten vorhandene Höchstdruckzusätze sind unter anderem schwefel- und/oder phosphor- und/oder halogenhaltige Stoffe, beispielsweise sulfuriertes Spermöl, sulfurierte Fette, Tritolylphosphat, chlorierte Paraffine oder äthoxylierte Phosphatester.

Die nachfolgenden Beispiele dienen der näheren Erläuterung der vorliegenden Erfindung. Teile und Prozentangaben sind Gewichtsteile und Gewichtsprozente, falls nicht anders angegeben. Die Ausgangsstoffe (Produkte aus Beispielen I bis IX) und deren Herstellung sind typischerweise in der britischen Patentschrift Nr. 1 490 287 beschrieben.

Herstellung der Ausgangsstoffe

Beispiel I

Man gibt 3 Teile in 7 Teilen Methanol gelöstes Kaliumhydroxyd zu einer Lösung von 170 Teilen 2-Undecanon in 200 Teilen t-Butanol. Man kühlt auf 5° C und versetzt daraufhin im Verlauf von drei Stunden mit einer Lösung von 106 Teilen Acrylnitril in 120 Teilen t-Butanol, wobei man die Temperatur bei 5° bis 10° C hält. Nach beendeter Zugabe läßt man den Ansatz sich auf Raumtemperatur erwärmen und rührt vier Stunden. Nach Zugabe von 5 Teilen konzentrierter Salzsäure (mit Wasser auf 15 Teile verdünnt), um den Katalysator zu neutralisieren, werden die Lösungsmittel und nicht umgesetztes Acrylnitril durch Destillation entfernt.

Der Rückstand wird zwischen Äther und Wasser verteilt, die ätherische Phase wird gewonnen und über Magnesiumsulfat getrocknet, der Äther wird entfernt, und der Rückstand wird im Vakuum destilliert.

Nach Entfernung von 5 Teilen nicht umgesetztes Ketons (Siedebereich 60°—65° C/0,6 Torr) erhält man eine bei 125°—135° C/0,5 Torr übergehende Fraktion (A) (38 Teile, was 17% Ausbeute auf Keton darstellt), die durch NMR und Elementaranalyse als 4-Acetyldodecanitril identifiziert wird:

$C_{14}H_{25}NO$
berechnet: C 75,28%; H 11,28%; N 6,27%
gefunden: C 75,56%; H 11,18%; N 6,54%

Eine zweite, bei 209°—212° C/0,3 Torr übergehende Fraktion (B) wird gewonnen (215 Teile, was 78% Ausbeute auf Keton darstellt), die durch NMR und Elementaranalyse als 4-n-Octyl-4-acetyl-pimelonitril identifiziert wird:

$C_{17}H_{28}N_2O$
berechnet: C 73,91%; H 10,14%; N 10,14%
gefunden: C 73,64%; H 10,41%; N 10,52%

Man gibt 138 Teile der Fraktion B in eine Lösung von 99 Teilen von Kaliumhydroxydplätzchen (85%ig) zu 600 Teilen Wasser und erhitzt unter Rühren zum Rückfluß. Nach acht Stunden Rückfluß wird die Lösung abgekühlt, mit konzentrierter Salzsäure auf pH 2 neutralisiert und der ausgefällte sirupöse Feststoff mit Äther extrahiert. Nach Waschen und Trocknen der Ätherlösung wird das Lösungsmittel entfernt, wobei ein fester Rückstand verbleibt, den man aus Essigester/Petroläther umkristallisiert, was 131 Teile (90% Ausbeute) 4-n-Octyl-4-acetylpimelinsäure (Schmelzpunkt 69°—71° C) ergibt, die durch NMR und Elementaranalyse identifiziert wird:

$C_{17}H_{30}O_5$
berechnet: C 64,94%; H 9,62%
gefunden: C 65,15%; H 9,71%

Beispiel II

Man gibt 22,3 Teile der im Beispiel I beschriebenen Fraktion A zu einer Lösung von 13,2 Teilen 85%igem Kaliumhydroxyd in 100 Teilen Wasser. Man erhitzt sechs Stunden zum Rückfluß, worauf die Lösung abgekühlt und mit konzentrierter Salzsäure auf pH 2 neutralisiert wird. Das ausgefallene Öl extrahiert man mit Äther, wäscht die Ätherlösung mit Wasser, trocknet über Magnesiumsulfat und entfernt das Lösungsmittel. Der Rückstand erstarrt beim Stehen und wird aus 40—60° Petroläther umkristallisiert, was 21,3 Teile (88% Ausbeute) 4-Acetyldodecansäure (Schmelzpunkt 36°—38° C) liefert, die durch NMR und Elementaranalyse identifiziert wird.

$C_{14}H_{26}O_3$
berechnet: C 69,38%; H 10,81%
gefunden: C 69,02%; H 10,58%

Bei Einarbeitung in eine wäßrige, mit Eisenmetallen in Berührung kommende Funktionsflüssigkeit weist 4-Acetyldodecansäure eine ausgezeichnete Korrosionsschutzwirkung auf.

### Beispiel III

Analog Beispiel I behandelt man 99 Teile 2-Tridecanon mit 53 Teilen Acrylnitril in t-Butanol. Nach Aufarbeitung liefert Destillation eine bei 159°–165°C/0,2 Torr übergehende Fraktion (A) (33 Teile, was 26% Ausbeute auf Keton darstellt), die durch NMR und Elementaranalyse als 4-Acetylmyristonitril identifiziert wird:

$C_{16}H_{29}NO$
berechnet: C 76,49%; H 11,55%; N 5,58%
gefunden: C 77,00%; H 11,53%; N 5,58%

Der Rückstand wird nicht weiter destilliert, jedoch durch NMR, Gas-Flüssigkeitschromatographie und Elementaranalyse als im wesentlichen (>95%) 4-n-Decyl-4-acetylpimelonitril (107 Teile, 70% Ausbeute) identifiziert.

$C_{19}H_{32}N_2O$
berechnet: C 75,00%; H 10,53%; N 9,21%
gefunden: C 74,52%; H 10,19%; N 9,41%

Der Rückstand wird gemäß Beispiel I hydrolysiert, was 107 Teile (75% Ausbeute) 4-n-Decyl-4-acetylpimelinsäure ergibt, die aus Essigester/Petroläther umkristallisiert wird.

$C_{19}H_{34}O_5$
berechnet: C 66,63%; H 10,01%; N 23,36%
gefunden: C 66,62%; H 9,81%; N 23,57%

### Beispiel IV

Auf die zuvor beschriebene Weise hydrolysiert man 25 Teile der Fraktion A gemäß Beispiel III, was 23 Teile (84% Ausbeute) 4-Acetylmyristinsäure ergibt:

$C_{16}H_{30}O_3$
berechnet: C 71,11%; H 11,11%
gefunden: C 71,23%; H 11,55%

In eine wäßrige Funktionsflüssigkeit eingearbeitete 4-Acetylmyristinsäure weist eine ausgezeichnete Korrosionshemmwirkung auf.

### Beispiel V

Man gibt 1,7 Teile in 4 Teilen Methanol gelöstes Kaliumhydroxyd zu einer Aufschlämmung von 100 Teilen Cyclododecanon in 100 Teilen t-Butanol. Man kühlt auf 10°C und versetzt im Verlauf von 1 ¼ Stunden tropfenweise mit der Lösung von 177 Teilen Acrylnitril in 60 Teilen t-Butanol, wobei man die Temperatur unterhalb 20°C hält. Nach beendeter Zugabe fällt das Cyanäthylzwischenprodukt beim Abkühlen auf Raumtemperatur spontan aus. Es wird abfiltriert, mit Äther gewaschen und nach den Methoden gemäß Beispiel I als im wesentlichen (>90%) 2,2,12-Tris-(2'-cyanäthyl)-cyclododecanon identifiziert (175 Teile; 93%; Schmelzpunkt 93°–98°C):

$C_{21}H_{31}N_3O$
berechnet: C 73,86%; H 9,15%; N 12,31%
gefunden: C 72,91%; H 8,81%; N 12,71%

Das Cyanäthylzwischenprodukt gibt man dann in eine Lösung von 135 Teilen Kaliumhydroxyd (85%ig) in 550 Teilen Wasser und erhitzt 17 Stunden zum Rückfluß. Nach dem Abkühlen versetzt man mit konzentrierter Salzsäure (ungefähr 200 Teile) bis auf pH 2, um das Produkt 2,2,12-Tris-(2'-carboxyäthyl)-cyclododecanon auszufällen. Dieses wird abfiltriert, gründlich mit warmen Wasser gewaschen und getrocknet, was 176 Teile (86,5% Ausbeute) vom Schmelzpunkt 187°–190°C liefert:

$C_{21}H_{34}O_7$
berechnet: C 63,29%; H 8,60%
gefunden: C 63,09%; H 8,27%

Weitere Beispiele unter Verwendung symmetrischer Dialkylketone und Anwendung der in den vorangehenden Beispielen dargelegten Arbeitsweisen sind in Tabelle I angeführt. Das Molverhältnis Keton : Acrylnitril für die Cyanäthylierungsstufe beträgt jeweils 1 : 3 in den Beispielen VI bis VIII.

Tabelle I

Tris-carboxyäthylalkanone

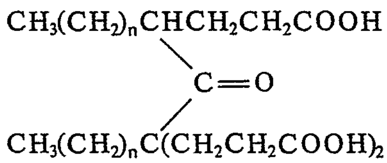

| Beispiel | Keton-Ausgangsstoff | n | Art des Produkts | Ausbeute (% auf Keton) | Elementaranalysen |
|---|---|---|---|---|---|
| VI | 6-Undecanon | 3 | Viskoser brauner Sirup | 91 | $C_{20}H_{34}O_7$<br>berechnet: C 62,15%; H 8,87%<br>gefunden: C 61,75%; H 8,98% |
| VII | 7-Tridecanon | 4 | Viskoser brauner Sirup | 89 | $C_{22}H_{38}O_7$<br>berechnet: C 63,74%; H 9,24%<br>gefunden: C 63,19%; H 9,14% |
| VIII | 9-Heptadecanon | 6 | Viskoser brauner Sirup | 74 | $C_{26}H_{46}O_7$<br>berechnet: C 66,35%; H 9,85%<br>gefunden: C 65,67%; H 9,48% |

## Beispiel IX

Man erhitzt 14,2 Teile 4-n-Octyl-4-acetylpimelinsäure und 3,5 Teile 1,2-propylenglykol in 50 Teilen Toluol zum Rückfluß bis zur Entfernung von 0,9 Teilen Wasser durch azeotrope Destillation. Man kühlt ab und entfernt das Toluol bei vermindertem Druck, was 16 Teile eines wachsartigen Feststoffs mit einer Säurezahl von 247 mg KOH/g ergibt.

## Beispiele 1 bis 3

Die Korrosionshemmwirkung der Produkte aus Beispiel I, II und V wird auf folgende Weise durch Versuche mit belüfteter Lösung in einer Flasche dargelegt, wobei man als Wasser ein künstliches weiches Wasser mit folgenden Eigenschaften verwendet:

| | |
|---|---|
| pH | 7,0 |
| Phenolphthaleinalkalinität | 0 |
| Gesamtalkalinität | 20 ppm als $CaCO_3$ |
| Gesamthärte | 35 ppm als $CaCO_3$ |
| Chloridion | 10 ppm als NaCl |
| Ryznev-Index | 10,4 |

5 cm × 1,5 cm große unlegierte Stahlabschnitte werden mit Bimsstein gescheuert, eine Minute in Salzsäure eingetaucht und dann abgespült, getrocknet und gewogen.

Dann löst man den gewünschten Anteil (50 oder 100 ppm) des zu prüfenden Korrosionshemmstoffs im Prüfwasser. Ein Stahlabschnitt wird in die Lösung gehängt und das Ganze in einer Flasche in einem Thermostaten bei 40°C aufbewahrt. Während der Lagerzeit leitet man 500 ml/Minute Luft in die Lösung, wobei der Stahlabschnitt gegen den Luftstrom abgeschirmt ist; Wasserverluste durch Verdunstung werden dauernd mit destilliertem Wasser aus einem Apparat mit konstanter Druckhöhe ersetzt.

Nach 48 Stunden wird der Stahlabschnitt herausgenommen, mit Bimsstein gescheuert, eine Minute in mit 1 Gew.-% Hexamin inhibierte Salzsäure eingetaucht und dann abgespült, getrocknet und zurückgewogen. Dabei beobachtet man, daß ein gewisser Gewichtsverlust eingetreten ist.

Gleichzeitig mit jeder Versuchsreihe wird ein Blindversuch durchgeführt, d. h. Eintauchen einer unlegierten Stahlprobe im Prüfwasser in Abwesenheit jedes möglichen Korrosionshemmstoffs. Die Korrosionsgeschwindigkeiten werden als Milligramm Gewichtsverlust/Quadratdezimeter/Tag (mdd) berechnet, doch werden die Ergebnisse der Einfachheit halber als Prozent Schutz angegeben, definiert wie folgt:

$$\%\,\text{Schutz} = \frac{\text{Korrosionsgeschwindigkeit im Blindversuch (in mdd)} - \text{Korrosionsgeschwindigkeit der Probe (in mdd)}}{\text{Korrosionsgeschwindigkeit im Blindversuch (in mdd)}}.$$

Die erhaltenen Ergebnisse sind in der folgenden Tabelle II angeführt.

Tabelle II

% Schutz in künstlichem, weichem Wasser

| Beispiel/ Vergleichs-beispiel (–) | Geprüfte Verbindung | Künstliches weiches Wasser 100 ppm |
|---|---|---|
| – | 4-Acetyl-4-n-amylpimelinsäure | 80 |
| – | 4-Acetyl-4-methylpimelinsäure | 68 |
| – | 1,1,1-Tris-(2'-carboxyäthyl)-aceton | +63 |
| – | 2,2,6,6-Tetra-(2'-carboxyäthyl)-cyclohexanon | 54 |
| – | 4-Methyl-2,2,6,6-tetra-(2'-carboxyäthyl)-cyclohexanon | 31 |
| – | 4-t-Butyl-2,2,6,6-tetra-(2'-carboxyäthyl)-cyclohexanon | 30 |
| – | 1,1,1-Tris-(2'-carboxyäthyl)-acetophenon | 27 |
| 1 | 4-n-Octyl-4-acetylpimelinsäure | 99 |
| 2 | 4-Acetyldodecansäure | 99 |
| 3 | 2,2,12-Tris-(2'-carboxyäthyl)-cyclododecanon | 96 |

Anmerkung: Das +-Zeichen bedeutet aggressive Korrosion (d. h. Korrosionswirkung, welche die in Abwesenheit irgendeiner Prüfverbindung erhaltene übersteigt).

Die Ergebnisse in Tabelle II belegen die ausgezeichnete, von den erfindungsgemäßen Zusammensetzungen gezeigte Korrosionshemmung gegenüber einer Zusammensetzung mit Verbindungen, die in ihrer chemischen Struktur den in Beispielen 1 bis 3 verwendeten Verbindungen sehr nahe verwandt sind.

## Beispiel 4 und 5

### Auswertung der Korrosionshemmung mit einer Laboratoriumswärmeaustauscheranlage

In dieser Anlage wird korrosives Wasser belüftet und über eine Anzahl Metallabschnitte im Kreislauf geführt, wobei es mittels Durchleiten durch ein erhitztes Stahlwärmeaustauscherrohr erhitzt wird. Nach einer geeigneten Prüfzeit werden die Metallabschnitte und das Wärmeaustauscherrohr untersucht und ihr Zustand beurteilt.

Im einzelnen besteht die Anlage aus einem geschlossenen Wasserkreislauf, der in dieser Reihenfolge aus den folgenden Teilen besteht:

20 Liter-Behälter
1 Liter-Behälter
Durchflußmesser
Abschnittskammer
Wärmeaustauscher
Kühler.

Korrosives Wasser im 20 Liter-Behälter wird mit ungefähr 5 Liter pro Minute durch eine Fritte eingeführte Druckluft belüftet und dann in den 1 Liter-Behälter gepumpt. Aus diesem Behälter wird es über den Durchflußmesser in die gläserne Abschnittskammer gepumpt, wo eine Anzahl rechteckiger Metallabschnitte von je 2,5 × 5,0 cm auf einem Plexiglasgestell angebracht sind. Dann fließt das Wasser durch den Wärmeaustauscher, der aus einem mit Kupferendstücken versehenen Stahlrohr von 1,58 cm Innendurchmesser besteht, welches mit einer 960 Watt Heizschlange bewickelt ist; aus dem Wärmeaustauscher fließt das Wasser durch den Kühler zurück in den 20 Liter-Behälter.

Eine Kreislaufdurchflußmenge von etwa 4,55 Liter pro Minute ergibt eine Geschwindigkeit von etwa 0,46 Meter pro Sekunde und eine Reynolds-zahl von 8500 im Wärmeaustauscher. Die Heizschlange erteilt dem Wärmeaustauscherrohr eine Oberflächentemperatur von etwa 60°C, und das Wasser tritt mit etwa 45°C aus, sodaß die Differenz über die Wärmeübergangsfläche etwa 15°C beträgt. Der Kühler wird so betrieben, daß er das Wasser auf etwa 40°C abkühlt, bevor es erneut in den Kreislauf eintritt.

Metallabschnitte werden mit Bimsstein gescheuert und dann wie folgt in Säure eingetaucht:

| Metall | Säure |
|---|---|
| Unlegierter Stahl | konzentrierte HCl mit Wasser 1 : 1 verdünnt, 1 Minute bei Raumtemperatur |
| Kupfer | ebenso |
| Messing | ebenso |
| Aluminium | 5% Phosphorsäure/2% Chromsäure, 5 Minuten bei 75°C |

Nach solchem Eintauchen werden die Abschnitte mit Wasser abgespült, getrocknet und gewogen; dann bringt man sie an einem Plexiglasgestell an, wobei zu beachten ist, daß keine Abschnitte sich gegenseitig berühren und daß sie von der das Gestell zusammenhaltenden Schraube isoliert sind. Das Wärmeaustauscherrohr wird mit Bimsstein gereinigt, in 1 : 1 mit Wasser verdünnte, konzentrierte Salzsäure getaucht und dann in Wasser abgespült und getrocknet.

Die Anlage wird zusammengebaut und durch Umpumpen von 1 : 1 mit Wasser verdünnter, konzentrierter Salzsäure und danach Durchspülen mit Leitungswasser für etwa eine halbe Stunde (im ganzen ungefähr 136,4 Liter) und Ablaufenlassen gründlich gereinigt. Die erforderliche Menge Zusatzstoffe, um die gewünschte Konzentrationen zu erhalten, wird in einen der Behälter gegeben, und die Anlage wird mit 22 Liter normiertem, korrosivem Manchester-prüfwasser gefüllt.

Die Pumpe wird zum Ansaugen gebracht und angelassen, und die Heizung wird eingeschaltet.

Die Hemmstoffkonzentration und der Wasserstand in der Anlage werden jeden Tag überprüft.

Nach drei Tagen und nochmals nach zehn Tagen wird das Wärmeaustauscherrohr herausgenommen, zerschnitten und untersucht. Die Prüfabschnitte werden entnommen, und die unlegierten Stahl- und Kupferabschnitte werden wie zuvor gereinigt, außer daß die Säure mit 1% Hexamin inhibiert ist, abgespült, getrocknet und zurückgewogen. Die Aluminiumproben werden gescheuert, getrocknet und zurückgewogen.

Mit den beobachteten Ergebnissen läßt sich die antikorrosive Wirkung des geprüften Hemmstoffs, unter Anwendung derselben Korrosionsgeschwindigkeitsgröße (in mdd) wie in Beispiel 1 bis 3 beurteilen.

Bei diesem Prüfungsverfahren erhält man die folgenden Ergebnisse:

Tabelle III

Beurteilung der Korrosionshemmung in der Laboratoriumsanlage

| Beispiel/ Vergleichsbeispiel (–) | Geprüfte Verbindung | Menge der geprüften Verbindung und Prüfungslänge | Korrosionsgeschwindigkeit (mdd) | | | | | |
|---|---|---|---|---|---|---|---|---|
| | | | unlegierter Stahl | Gußeisen | Al | Cu | Messing | Wärmeaustauscher aus unlegiertem Stahl |
| – | ohne | – | 140,1 | 180,2 | 5,8 | 3,7 | 4,8 | 420,6 |
| – | 4-Acetylnonansäure | 100 ppm 3 Tage; 25 ppm 10 Tage | 84,9 | 90,8 | 0,0 | 0,0 | 0,0 | 283,1 |
| 4 | 4-n-Octyl-4-acetylpimelinsäure | 100 ppm 3 Tage; 20 ppm 10 Tage | 2,1 | 11,8 | 0,6 | 0,2 | 0,3 | 36,5 |
| 5 | 2,2,12-Tris-(2'-carboxyäthyl)-cyclodecanon | 100 ppm 3 Tage; 25 ppm 10 Tage | 7,4 | 110,6 | 2,1 | 0,3 | 0,5 | 90,8 |

Beispiel 6 bis 13

Die Korrosionsbeständigkeit verschiedener wäßriger, erfindungsgemäßer Schneidölzusammensetzungen wird nach der folgenden Arbeitsweise beurteilt, welche eine Abänderung der Institute of Petroleum-prüfung 287 darstellt.

Man löst 1 Gramm der zu prüfenden Verbindung in Wasser (entweder entsalztes Wasser oder ein normiertes hartes Wasser) und gibt so viel Triäthanolamin (TEA) dazu, daß die Lösung einen pH-Wert von ungefähr 9 aufweist. Ein 2-ml-Anteil dieser Lösung wird dann unter den in der IP 287-Prüfvorschrift angegebenen Bedingungen mit auf einem Filterpapier befindlichen Metallspänen in Berührung gebracht.

Die visuelle Beurteilung des Filterpapiers nach der Behandlung erfolgt gemäß folgenden Richtlinien:

| Rostgrad | Bewertung |
|---|---|
| Kein Rost | 0 |
| ≤5 kleine Flecken | T (Spur) |
| ≤10% der Fläche gerostet | M (mäßig) |
| >10% der Fläche gerostet | S (stark) |

Tabelle IV

Korrosionsbeständigkeit in wäßrigen Schneidflüssigkeiten

| Beispiel/ Vergleichs- beispiel (—) | Geprüfte Verbindung | % TEA pro 1% geprüfte Ver- bindung | pH | IP 287-Prüfdaten Verdünnungs- verhältnis*) | Rost (entsalztes Wasser) | Rost (hartes Wasser) |
|---|---|---|---|---|---|---|
| — | 4-Acetyl-4-n-amyl- pimelinsäure | 3,0 | 8,6 | 1 : 50 | M | S |
| | | | | 1 : 100 | S | — |
| | | | | 1 : 200 | S | — |
| — | 4-Acetyl-4-methyl- pimelinsäure | 5,75 | 8,9 | 1 : 30 | S | S |
| — | 1,1,1-Tris-(2'-carboxy- äthyl)-aceton | 5,25 | 8,8 | 1 : 32 | S | S |
| 6 | 4-n-Octyl-4-acetyl- pimelinsäure | 3,4 | 9,0 | 1 : 46 | 0 | 0 |
| | | | | 1 : 92 | 0 | 0 |
| | | | | 1 : 184 | 0 | M |
| 7 | 2,2,10-Tris-(2'-carboxy- äthyl)-cyclodecanon | 6,5 | 9,0 | 1 : 26 | 0 | 0 |
| | | | | 1 : 52 | 0 | 0 |
| | | | | 1 : 104 | 0 | M |
| 8 | 4-Acetyldodecansäure | 4,1 | 9,0 | 1 : 40 | 0 | 0 |
| | | | | 1 : 160 | 0 | T-M |
| | | | | 1 : 320 | 0 | — |
| 9 | 4-n-Decyl-4-acetyl- pimelinsäure | 4,7 | 8,9 | 1 : 34 | 0 | 0 |
| | | | | 1 : 68 | 0 | T |
| | | | | 1 : 136 | 0 | M |
| | | | | 1 : 272 | M | S |
| 10 | 2,12-Bis-(2'-carboxy- äthyl)-cyclododecanon | 5,9 | 9,0 | 1 : 28 | 0 | 0 |
| | | | | 1 : 112 | 0 | M |
| | | | | 1 : 224 | 0 | — |
| 11 | Produkt aus Beispiel VI | 5,5 | 9,0 | 1 : 30 | 0 | 0 |
| | | | | 1 : 120 | 0 | T |
| | | | | 1 : 240 | M | S |
| 12 | Produkt aus Beispiel VII | 5,3 | 9,0 | 1 : 32 | 0 | 0 |
| | | | | 1 : 128 | 0 | T |
| | | | | 1 : 256 | T | — |
| 13 | Produkt aus Beispiel VIII | 4,9 | 9,1 | 1 : 34 | 0 | 0 |
| | | | | 1 : 68 | 0 | M |
| | | | | 1 : 136 | T | — |
| Kontrolle | ohne Hemmstoff | — | — | 1 : 20**) | 0 | S |
| | | | | 1 : 50 | M | S |
| | | | | 1 : 100 | S | S |

*) Bezogen auf die gesamte Menge der geprüften Verbindung und des Triäthanolamins zur Menge Wasser.

**) Das Verdünnungsverhältnis bezieht sich auf die Menge Triäthanolamin zur Menge Wasser.

Bezüglich der Prüfergebnisse in Tabelle IV ist zu sagen, daß wegen der Gengenwart von TEA im Überschuß über das stöchiometrische Äquivalent der zu prüfenden Säure diese als Triäthanolaminsalz vorliegen.

Beispiel 14 bis 19

Die Korrosionsbeständigkeit verschiedener, erfindungsgemäßer Ölzusammensetzungen wird gemäß der Arbeitsweise ASTM D665A beurteilt.

Bei diesem Prüfverfahren wird eine nach vorgeschriebener Methode vorgereinigte, unlegierte Stahlprobe unter Rühren in 300 ml Öl, das den zu prüfenden Hemmstoff enthält, eingetaucht und 30 Minuten bei 60°C gehalten. Dann gibt man 30 ml entsalztes Wasser dazu und rührt noch 24 Stunden bei 60°C weiter.

Am Ende dieses Zeitraums wird die Metallprobe visuell gegenüber folgendem Standard untersucht:

| Rostgrad | Bewertung |
|---|---|
| Sauber und glänzend | C + B |
| leicht gerostet (<5 kleine Flecken) | L (leicht) |
| ≤10% der Fläche gerostet | M (mäßig) |
| >10% der Fläche gerostet | S (stark) |

Tabelle V

Korrosionsbeständigkeit in Ölzusammensetzungen

| Beispiel/ Vergleichsbeispiel (—) | geprüfte Verbindung | ASTM-D665A-Prüfung Konzentration (%) | Rostgrad |
|---|---|---|---|
| — | 4-n-Acetyl-4-n-amylpimelinsäure | 0,05<br>0,025 | M<br>S |
| 14 | 4-n-Octyl-4-acetylpimelinsäure | 0,05<br>0,013 | C + B<br>M |
| 15 | 4-Acetyldodecansäure | 0,05<br>0,013 | C + B<br>C + B |
| 16 | 4-n-Decyl-4-acetylpimelinsäure | 0,05 | C + B |
| 17 | 4-Acetylmyristinsäure | 0,05 | C + B |
| 18 | Produkt aus dem Halbester aus Beispiel 1 plus Propylen-1,2-glykol | 0,025<br>0,013<br>0,006 | C + B<br>C + B<br>C + B |
| 19 | 3 : 2-Gemisch der Produkte aus Beispiel 25 und 26 | 0,013<br>0,006 | C + B<br>C + B |

Ein Kontrollversuch (ohne Hemmstoff) ergab äußerst starkes Rosten.

**Patentansprüche**

1. Zusammensetzungen, dadurch gekennzeichnet, daß sie aus einer mit Eisenmetallen in Berührung kommenden Funktionsflüssigkeit und, als Korrosionshemmstoff, einer Verbindung der Formel

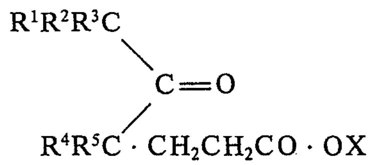

(I)

worin X für H ein Alkali- oder Erdalkalimetall, $NH_4$ oder den Rest eines protonierten Amins steht, oder die Gruppe $-OX$ den Rest eines Alkanols mit 1 bis 20 Kohlenwasserstoffatomen oder eines Di-, Tri- oder Tetraols mit 2 bis 12 Kohlenstoffatomen darstellt, $R^1$, $R^2$ und $R^4$ gleich oder verschieden sind und je für H oder $-CH_2CH_2COOX$ stehen, wobei X seine obige Bedeutung hat, $R^3$ und $R^5$ gleich oder verschieden sind und je für H oder $-CH_2CH_2COOX$ stehen, wobei X seine obige Bedeutung hat, bzw. $R^3$ und $R^5$ eine geradkettige oder verzweigte Alkylgruppe mit 3 bis 16 Kohlenstoffatomen darstellen oder gegebenenfalls $R^3$ und $R^5$ zusammen mit dem Kohlenstoffatom, an das sie gebunden sind, einen Cycloalkanonring mit 8 bis 15 Kohlenstoffatomen bilden, bestehen, mit der Bedingung, daß $R^4$ und $R^5$ gleichzeitig den Rest $-CH_2CH_2COOX$ nicht bedeuten können, wenn $R^1$, $R^2$ und $R^3$ gleichzeitig Wasserstoff sind, und daß $R^5$ keine Alkylgruppe mit 3 bis 5 Kohlenstoffatomen darstellen kann, wenn $R^1$, $R^2$ und $R^3$ gleichzeitig Wasserstoff und $R^4$ den Rest $-CH_2CH_2COOX$ oder Wasserstoff sind, wobei X seine obige Bedeutung hat.

2. Zusammensetzung nach Anspruch 1, dadurch gekennzeichnet, daß X für Na, K oder $NH_4$ steht.

3. Zusammensetzung nach Anspruch 1 oder 2, dadurch gekennzeichnet, daß $R^3$ oder $R^5$ für eine geradkettige oder verzweigte Alkylgruppe mit 7 bis 12 Kohlenstoffatomen steht.

4. Zusammensetzung nach einem der vorhergehenden Ansprüche, dadurch gekennzeichnet, daß der Anteil der Verbindung der Formel I 0,001 bis 5 Gew.-% beträgt, bezogen auf das Gesamtgewicht der Zusammensetzung.

5. Zusammensetzung nach einem der vorhergehenden Ansprüche, dadurch gekennzeichnet, daß als Grundzusammensetzung ein wäßriges System vorliegt und der Hemmstoff der Formel

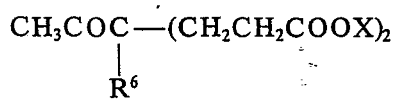

(II)

worin X die in Anspruch 1 angegebene Bedeutung hat und $R^6$ für eine Alkylgruppe mit 6 bis 16 Kohlenstoffatomen steht, oder deren Monocarboxyäthylanalog entspricht.

6. Zusammensetzung nach Anspruch 5, dadurch gekennzeichnet, daß X für H, Na oder K und $R^6$ für eine Alkylgruppe mit 7 bis 12 Kohlenstoffatomen stehen.

7. Zusammensetzung nach Anspruch 6, dadurch gekennzeichnet, daß X für H, Na oder K und $R^6$ für eine Octylgruppe stehen.

8. Zusammensetzung nach einem der Ansprüche 1 bis 4, dadurch gekennzeichnet, daß als Grundzusammensetzung ein wäßriges System vorliegt und der Hemmstoff der Formel

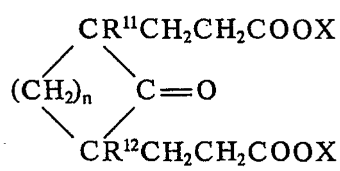

(IV)

worin X die in Anspruch 1 angegebene Bedeutung hat, $R^{11}$ und $R^{12}$ gleich oder verschieden sind und je für H oder $-CH_2CH_2COOX$ stehen, wobei X seine obige Bedeutung hat, und n eine ganze Zahl von 5 bis 12 ist; entspricht.

9. Zusammensetzung nach Anspruch 8, dadurch gekennzeichnet, daß n eine ganze Zahl von 9 bis 11 ist.

10. Zusammensetzung nach Anspruch 8 oder 9, dadurch gekennzeichnet, daß X für H, Na oder K, $R^{11}$ für H und $R^{12}$ für $-CH_2CH_2COOX$ stehen, worin X die in Anspruch 1 angegebene Bedeutung hat, und n 9 ist.

11. Zusammensetzung nach einem der Ansprüche 1 bis 4, dadurch gekennzeichnet, daß als Grundzusammensetzung eine Metallbearbeitungsflüssigkeit vorliegt und der Hemmstoff der Formel

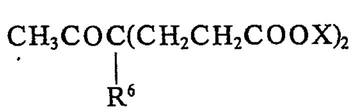

(II)

worin X und $R^6$ die in Anspruch 6 angegebene Bedeutung haben, entspricht.

12. Zusammensetzung nach Anspruch 11, dadurch gekennzeichnet, daß X für H, Diäthanolamin, Triäthanolamin oder Morpholin und $R^6$ für eine Octylgruppe stehen.

13. Zusammensetzung nach Anspruch 12, dadurch gekennzeichnet, daß X für Triäthanolamin steht.

14. Zusammensetzung nach einem der Ansprüche 1 bis 4, dadurch gekennzeichnet, daß als Grundzusammensetzung eine Metallbearbeitungsflüssigkeit vorliegt und der Hemmstoff der Formel

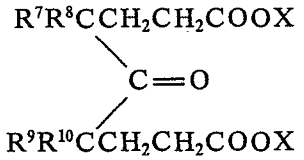 (III)

worin X die in Anspruch 1 angegebene Bedeutung hat, $R^8$ und $R^9$ gleich oder verschieden sind und je für H oder $-CH_2CH_2COOX$ stehen, wobei X die in Anspruch 1 angegebene Bedeutung hat, sowie $R^7$ und $R^{10}$ gleich oder verschieden sind und je für eine Alkylgruppe mit 3 bis 10 C-Atomen stehen, entspricht.

15. Zusammensetzung nach Anspruch 14, dadurch gekennzeichnet, daß X für H, Diäthanolamin, Triäthanolamin oder Morpholin, $R^8$ für H sowie $R^7$ und $R^{10}$ je für eine Amylgruppe stehen.

16. Zusammensetzung nach einem der Ansprüche 1 bis 4, dadurch gekennzeichnet, daß als Grundzusammensetzung eine Metallbearbeitungsflüssigkeit vorliegt und der Hemmstoff der Formel

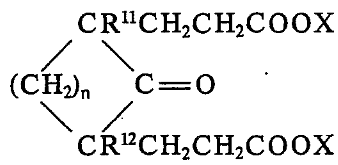 (IV)

worin X die in Anspruch 1 und $R^{11}$, $R^{12}$ und n die in Anspruch 8 angegebenen Bedeutungen haben, entspricht.

17. Zusammensetzung nach Anspruch 16, dadurch gekennzeichnet, daß X für H, Diäthanolamin, Triäthanolamin oder Morpholin, $R^{11}$ für H und $R^{12}$ für $CH_2CH_2COOX$ stehen, wobei X die in Anspruch 1 angegebene Bedeutung hat, und n 9 ist.

18. Zusammensetzung nach Anspruch 17, dadurch gekennzeichnet, daß X für Triäthanolamin steht.

19. Zusammensetzung nach einem der Ansprüche 1 bis 4, dadurch gekennzeichnet, daß eine Funktionsflüssigkeit auf Ölgrundlage vorliegt und $R^1$, $R^2$ und $R^3$ je für H stehen, $R^4$ die in Anspruch 1 angegebene Bedeutung hat, $R^5$ eine Alkylgruppe mit 8 bis 16 Kohlenstoffatomen darstellt und X H oder ein protoniertes Amin oder, falls $R^4$ $-CH_2CH_2COOH$ ist, eine Alkyl- oder Hydroxyalkylgruppe bedeutet.

20. Zusammensetzung nach einem der Ansprüche 1 bis 4, dadurch gekennzeichnet, daß eine Funktionsflüssigkeit auf Ölgrundlage vorliegt und $R^1$ H ist, $R^2$ und $R^4$ entweder beide für H oder eines für H und das andere für $-CH_2CH_2COOX$ stehen, wobei X die in Anspruch 1 angegebene Bedeutung hat, $R^3$ und $R^5$ je Alkyl mit 5 bis 16 Kohlenstoffatomen darstellen und X H oder ein protoniertes Amin oder, falls $R^2$ oder $R^4$ $-CH_2CH_2COOH$ ist, eine Alkyl- oder Hydroxyalkylgruppe bedeutet.

21. Zusammensetzung nach Anspruch 20, dadurch gekennzeichnet, daß $R^3$ und $R^5$ je für Alkyl mit 8 bis 16 Kohlenstoffatomen stehen.

22. Zusammensetzung nach einem der Ansprüche 1 bis 4, dadurch gekennzeichnet, daß eine Funktionsflüssigkeit auf Ölgrundlage vorliegt und $R^1$ H ist, $R^2$ und $R^4$ entweder beide für H oder eines für H und das andere für $-CH_2CH_2COOX$ stehen, $R^3$ und $R^5$ zusammen die Gruppe $-(CH_2)_m-$ darstellen, worin m eine ganze Zahl von 9 bis 12 ist, und X H oder ein protoniertes Amin oder, falls $R^2$ oder $R^4$ $-CH_2CH_2COOH$ ist, eine Alkyl- oder Hydroxylalkylgruppe bedeutet.

23. Zusammensetzung nach einem der Ansprüche 1 bis 22, dadurch gekennzeichnet, daß der Hemmstoff der Formel I zusammen mit einem oder mehreren weiteren Zusatzstoffen verwendet wird.

**Claims**

1. Compositions, characterised in that they consist of a functional fluid coming into contact with ferrous metals and, as corrosion inhibitor, a compound of the formula

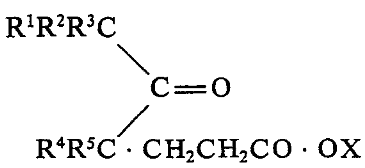 (I)

wherein X is H, an alkali metal or alkaline-earth metal, $NH_4$ or the radical of a protonated amine, or the group $-OX$ is the radical of an alkanol having 1 to 20 carbon atoms or of a di-, tri- or tetra-ol having 2 to 12 carbon atoms; $R^1$, $R^2$ and $R^4$ are identical or different and are each H or $-CH_2CH_2COOX$, in which X has the above meaning, $R^3$ and $R^5$ are identical or different and are each H or $-CH_2CH_2COOX$, in which X has the above meaning, or $R^3$ or $R^5$ is a straight-chain or branched-chain alkyl group having 3 to 16 carbon atoms, or $R^3$ and $R^5$ together with the carbon atom to which they are attached can form a cycloalkanone ring having 8 to 15 carbon atoms, with the proviso that $R^4$ and $R^5$ cannot simultaneously be the group $-CH_2CH_2COOX$ when $R^1$, $R^2$ and $R^3$ are simultaneously hydrogen, and that $R^5$ cannot be an alkyl group having 3 to 5 carbon atoms when $R^1$, $R^2$ and $R^3$ are simultaneously hydrogen and $R^4$ is the group $-CH_2CH_2COOX$ or hydrogen, in which X has the above meaning.

2. Composition according to claim 1, characterised in that X is Na, K or $NH_4$.

3. Composition according to claim 1 or 2, characterised in that $R^3$ or $R^5$ is a straight-chain or branched-chain alkyl group having 7 to 12 carbon atoms.

4. Composition according to any one of the preceding claims, characterised in that the proportion of the compound of the formula I is 0.001 to 5% by weight, relative to the total weight of the composition.

5. Composition according to any one of the preceding claims, characterised in that the base composition is in the form of an aqueous system, and the inhibitor corresponds to the formula

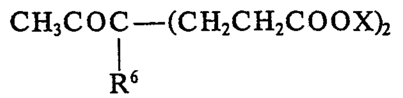

(II)

wherein X has the meaning defined in claim 1, and $R^6$ is an alkyl group having 6 to 16 carbon atoms, or the monocarboxyethyl analogue thereof.

6. Composition according to claim 5, characterised in that X is H, Na or K, and $R^6$ is an alkyl group having 7 to 12 carbon atoms.

7. Composition according to claim 6, characterised in that X is H, Na or K, and $R^6$ is an octyl group.

8. Composition according to any one of claims 1 to 4, characterised in that the base composition is an aqueous system, and the inhibitor corresponds to the formula

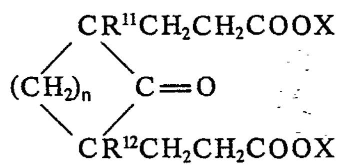

(IV)

wherein X is as defined in claim 1, $R^{11}$ and $R^{12}$ are identical or different and are each H or $-CH_2CH_2COOX$, in which X has the above meaning, and n is an integer from 5 to 12.

9. Composition according to claim 8, characterised in that n is an integer from 9 to 11.

10. Composition according to claim 8 or 9, characterised in that X is H, Na or K, $R^{11}$ is H, and $R^{12}$ is $-CH_2CH_2COOX$, in which X has the meaning defined in claim 1, and n is 9.

11. Composition according to any one of claims 1 to 4, characterised in that the base composition is a metal working fluid, and the inhibitor corresponds to the formula

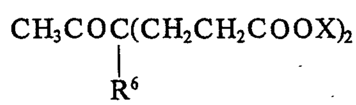

(II)

wherein X and $R^6$ have the meanings defined in claim 6.

12. Composition according to claim 11, characterised in that X is H, diethanolamine, triethanolamine or morpholine, and $R^6$ is an octyl group.

13. Composition according to claim 12, characterised in that X is triethanolamine.

14. Composition according to any one of claims 1 to 4, characterised in that the base composition is a metal working fluid, and the inhibitor corresponds to the formula

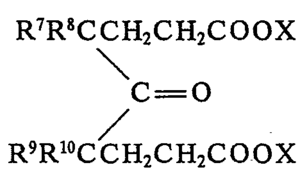

(III)

wherein X has the meaning defined in claim 1, $R^8$ and $R^9$ are identical or different and are each H or $-CH_2CH_2COOX$, in which X has the meaning defined in claim 1, and $R^7$ and $R^{10}$ are identical or different and are each an alkyl group having 3 to 10 C atoms.

15. Composition according to claim 14, characterised in that X is H, diethanolamine, triethanolamine

or morpholine, $R^8$ is H, and $R^7$ and $R^{10}$ are each an amyl group.

16. Composition according to any one of claims 1 to 4, characterised in that the base composition is a metal working fluid, and the inhibitor corresponds to the formula

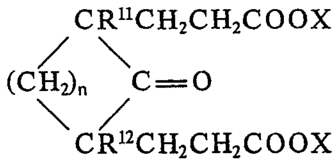

(IV)

wherein X has the meaning defined in claim 1, and $R^{11}$, $R^{12}$ and n have the meanings defined in claim 8.

17. Composition according to claim 16, characterised in that X is H, diethanolamine, triethanolamine or morpholine, $R^{11}$ is H, and $R^{12}$ is $CH_2CH_2COOX$, in which X has the meaning defined in claim 1, and n is 9.

18. Composition according to claim 17, characterised in that X is triethanolamine.

19. Composition according to any one of claims 1 to 4, characterised in that the composition is an oilbased functional fluid, and $R^1$, $R^2$ and $R^3$ are each H, $R^4$ has the meaning defined in claim 1, $R^5$ is an alkyl group having 8 to 16 carbon atoms, and X is H or a protonated amine, or, when $R^4$ is $-CH_2CH_2COOH$, X is an alkyl or hydroxyalkyl group.

20. Composition according to any one of claims 1 to 4, characterised in that the composition is an oilbased functional fluid, and $R^1$ is H, $R^2$ and $R^4$ are each H or one is H and the other is $-CH_2CH_2COOX$, in which X has the meaning defined in claim 1, $R^3$ and $R^5$ are each alkyl having 5 to 16 carbon atoms, and X is H or a protonated amine or, when $R^2$ or $R^4$ is $-CH_2CH_2COOH$, X is an alkyl or hydroxyalkyl group.

21. Composition according to claim 20, characterised in that $R^3$ and $R^5$ are each alkyl having 8 to 16 carbon atoms.

22. Composition according to any one of claims 1 to 4, characterised in that the composition is an oilbased functional fluid, and $R^1$ is H, $R^2$ and $R^4$ are each H, or one is H and the other is $-CH_2CH_2COOX$, $R^3$ and $R^5$ together are the group $-(CH_2)_m-$, wherein m is an integer from 9 to 12, and X is H or a protonated amine, or, when $R^2$ or $R^4$ is $-CH_2CH_2COOH$, X is an alkyl or hydroxyalkyl group.

23. Composition according to any one of claims 1 to 22, wherein the inhibitor of the formula I is used together with one or more further additives.

**Revendications**

1. Compositions caractérisées an ce qu'elles comprennent un liquide fonctionnel entrant en contact avec des métaux ferreux et, comme inhibiteur de corrosion, un composé répondant à la formule (I):

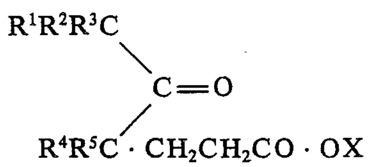

(I)

dans laquelle:

X représente H, un métal alcalin, un métal alcalinoterreux, $NH_4$ ou le radical d'une amine protonée, ou encore $-OX$ représente le radical d'un alcanol contenant de 1 à 20 atomes de carbone ou d'un diol, triol ou tétrol contenant de 1 à 12 atomes de carbone,

$R^1$, $R^2$ et $R^4$ représentent chacun, indépendamment les uns des autres, H ou un radical $-CH_2CH_2COOX$ dans lequel X a la signification qui vient de lui être donnée, et

$R^3$ et $R^5$ représentent chacun, indépendamment l'un de l'autre, H ou un radical $-CH_2CH_2COOX$ dans lequel X a la signification qui vient de lui être donnée, ou encore $R^3$ ou $R^5$ représente un radical alkyle, linéaire ou ramifié, contenant de 3 à 16 atomes de carbone, ou, éventuellement, $R^3$ et $R^5$ forment ensemble, et avec l'atome de carbone auquel ils sont liés, un noyau de cycloalcanone contenant de 8 à 15 atomes de carbone,

avec la condition que $R^4$ et $R^5$ ne représentent pas chacun, en même temps, un radical $-CH_2CH_2COOX$ dans le cas où $R^1$, $R^2$ et $R^3$ sont chacun l'hydrogène, et que $R^5$ ne représente pas un alkyle en $C_3-C_5$ dans le cas où $R^1$, $R^2$ et $R^3$ sont chacun l'hydrogène et $R^4$ est l'hydrogène ou un radical $-CH_2CH_2COOX$ dans lequel X a, là encore, la signification qui lui a été attribuée plus haut.

2. Composition selon la revendication 1, caractérisée en ce que X représente Na, K ou $NH_4$.

3. Composition selon l'une des revendications 1 et 2, caractérisée en ce que $R^3$ ou $R^5$ représente un radical alkyle, linéaire ou ramifié, contenant de 7 à 12 atomes de carbone.

4. Composition selon l'une quelconque des revendications précédentes, caractérisée en ce que la proportion du composé de furmule (I) est comprise entre 0,001 et 5% en poids par rapport au poids total de la composition.

5. Composition selon l'une quelconque des revendications précédentes, caractérisée en ce qu'elle a pour base un système aqueux et en ce que l'inhibiteur qu'elle contient répond à la formule (II):

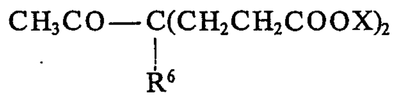

(II)

dans laquelle X a la signification donnée à la revendication 1 et $R^6$ représente un radical alkyle contenant de 6 à 16 atomes de carbone, ou est l'analogue mono-carboxyéthylique correspondant.

6. Composition selon la revendication 5 caractérisée en ce que X représente H, Na ou K et $R^6$ représente un radical alkyle contenant de 7 à 12 atomes de carbone.

7. Composition selon la revendication 6 caractérisée en ce que X représente H, Na ou K et $R^6$ un radical octyle.

8. Composition selon l'une quelconque des revendications 1 à 4, caractérisée en ce qu'elle a pour base un système aqueux et en ce que l'inhibiteur qu'elle contient répond à la formule (IV):

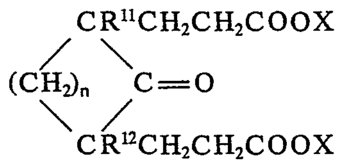

(IV)

dans laquelle X a la signification donnée à la revendication 1, $R^{11}$ et $R^{12}$ représentent chacun, indépendamment l'un de l'autre, H ou un radical $-CH_2CH_2COOX$ dans lequel X a la signification précédemment donnée, et n désigne un nombre entier de 5 à 12.

9. Composition selon la revendication 8, caractérisée en ce que n est un nombre entier de 9 à 11.

10. Composition selon l'une des revendications 8 et 9, caractérisée en ce que X représente H, Na ou K, $R^{11}$ représente H, $R^{12}$ représente un radical $-CH_2CH_2COOX$ dans lequel X a la signification donnée à la revendication 1, et n est égal à 9.

11. Composition selon l'une quelconque des revendications 1 à 4, caractérisée en ce que sa base est un liquide pour usinage de métaux et en ce que l'inhibiteur qu'elle contient répond à la formule (II):

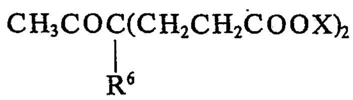

(II)

dans laquelle X et $R^6$ ont les significations données à la revendication 6.

12. Composition selon la revendication 11, caractérisée en ce que X représente H, la diéthanolamine, la triéthanolamine ou la morpholine et $R^6$ représente un radical octyle.

13. Composition selon la revendication 12 caractérisée en ce que X représente la triéthanolamine.

14. Composition selon l'une quelconque des revendications 1 à 4, caractérisée en ce que sa base est un liquide pour l'usinage des métaux et en ce que l'inhibiteur qu'elle contient répond à la formule (III):

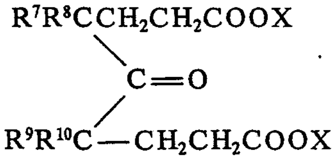

(III)

dans laquelle X a la signification donnée à la revendication 1, $R^8$ et $R^9$ représentent chacun, indépendamment l'un de l'autre, H ou un radical $-CH_2CH_2COOX$ dans lequel X a la signification donnée à la revendication 1, et $R^7$ et $R^{10}$ représentent chacun, indépendamment l'un de l'autre, un radical alkyle contenant de 3 à 10 atomes de carbone.

15. Composition selon la revendication 14, caractérisée en ce que X représente H, la diéthanolamine, la triéthanolamine ou la morpholine, $R^8$ représente H, et $R^7$ et $R^{10}$ représentent chacun un radical pentyle.

16. Composition selon l'une quelconque des revendications 1 à 4, caractérisée en ce que sa base est un liquide pour l'usinage des métaux et en ce que l'inhibiteur qu'elle contient répond à la formule (IV):

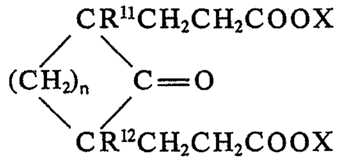

(IV)

dans laquelle X a la signification donnée à la revendication 1, et $R^{11}$, $R^{12}$ et n ont les significations données à la revendication 8.

17. Composition selon la revendication 16 caractérisée en ce que X représente H, la diéthanolamine, la triéthanolamine ou la morpholine, $R^{11}$ représente H, $R^{12}$ représente un radical $-CH_2CH_2COOX$ dans lequel X a la signification donnée à la revendication 1, et n est égal à 9;

18. Composition selon la revendication 17 caractérisée en ce que X représente la triéthanolamine.

19. Composition selon l'une quelconque des revendications 1 à 4, caractérisée en ce que son liquide fonctionnel est à base d'huiles et en ce que $R^1$, $R^2$ et $R^3$ représentent chacun H, $R^4$ a la signification donnée à revendication 1, $R^5$ représente un radical alkyle contenant de 8 à 16 atomes de carbone et X représente H, une amine protonée ou, dans le cas où $R^4$ est un radical $-CH_2CH_2COOH$, un radical alkyle ou hydroxyalkyle.

20. Composition selon l'une quelconque des revendications 1 à 4, caractérisée en ce que son liquide fonctionnel est à base d'huiles et en ce que $R^1$ représente H, $R^2$ et $R^4$ représentent soit chacun un même temps H, soit l'un H et l'autre un radical $-CH_2CH_2COOX$ dans lequel X a la signification donnée à la revendication 1, $R^3$ et $R^5$ représentent chacun un radical alkyle contenant de 5 à 16 atomes de carbone, et X représente H, une amine protonée ou, dans le cas où $R^2$ ou $R^4$ représente $-CH_2CH_2COOH$, un radical alkyle ou hydroxyalkyle.

21. Composition selon la revendication 20, caractérisée en ce que $R^3$ et $R^5$ représentent chacun un radical alkyle contenant de 8 à 16 atomes de carbone.

22. Composition selon l'une quelconque des revendications 1 à 4, caractérisée en ce que son liquide fonctionnel est à base d'huiles et en ce que $R^1$ représente H, $R^2$ et $R^4$ représentent soit chacun en même temps H, soit l'un H et l'autre un radical $-CH_2CH_2COOX$, $R^3$ et $R^5$ forment ensemble un radical $-(CH_2)_m-$ dans lequel m désigne un nombre entier de 9 à 12, et X représente H, une amine protonée ou, dans le cas où $R^2$ ou $R^4$ représente $-CH_2CH_2COOH$, un radical alkyle ou hydroxyalkyle.

23. Composition selon l'une quelconque des revendications 1 à 22, caractérisée en ce que l'inhibiteur de formule (I) est utilisé en association avec un ou plusieurs additifs supplémentaires.